# EUROPEAN PATENT APPLICATION

(11) **EP 3 498 271 A1**
(43) Date of publication of application: **19.06.2019**
(21) Application number: 18215914.5
(22) Date of filing: 02.05.2012
(51) Int. Cl.: A61K 31/198, A61P 3/10, A61P 3/00, A61K 31/19, A61K 45/06, A61K 31/4015, A61K 31/197, A61K 31/40, A61K 9/00, A61K 9/02, A61K 9/28

(54) **COMPOSITION AND METHOD FOR TREATMENT OF DIABETES**

(30) Priority: 02.05.2011 US 201161481268 P
(62) Divisional of application: 12779478.2
(71) Applicant: Biokier, Inc., Chapel Hill, NC 27514 (US)
(72) Inventor: Szewczyk, Jerzy Ryszard, Chapel Hill, NC North Carolina 27514 (US)
(74) Representative: Barker Brettell LLP

(57) **Abstract**

The present invention relates to a method of treating diabetes type II by delivery of butyric acid, bile acid, long chain fatty acid or glutamine to the colon by bypassing the upper digestive tract. The composition is combined either by the same or different route of administration with a DPP-IV inhibitor such as vildagliptin.

## Description

This application claims priority of US provisional application number 61/481,268 filed on May 2, 2011 and is included herein in its entirety by reference.

### COPYRIGHT NOTICE

A portion of the disclosure of this patent contains material that is subject to copyright protection. The copyright owner has no objection to the reproduction by anyone of the patent document or the patent disclosure as it appears in the Patent and Trademark Office patent files or records, but otherwise reserves all copyright rights whatsoever.

### BACKGROUND OF THE INVENTION

### Field of the Invention

The present invention relates to a novel method and composition method for treating diabetes, metabolic syndrome, hypertriglyceridemia, polycystic ovarian syndrome (PCOS) and obesity. In particular, the present invention relates to the treatment of diabetes, metabolic syndrome, hypertriglyceridemia, polycystic ovarian syndrome (PCOS) and obesity by delivering specific, naturally occurring compounds to the lower gut in combination with a dipeptidyl peptidase inhibitor in a manner that induces secretion of and prevents degradation of the secreted endogenous gut hormones.

### Description of Related Art

The use of bariatric surgery is a popular and very effective method of treating obesity.

Diabetes mellitus is a worldwide health threat of increasing magnitude and is considered a major health risk in both developed and developing countries. Type II diabetes accounts for the vast majority of the cases involving diabetes and accounts suggest it is the seventh leading cause of death in the United States. It appears that the major contributing factor to the incidence of Type II diabetes is being overweight. In the United States alone, it is estimated that over 17.6 million individuals suffer from diabetes and it is estimated that an additional 5.7 million individuals are unaware they have diabetes. In addition, there are about 57 million Americans who are considered pre-diabetic.

Type II diabetes is also known as non-insulin dependent diabetes mellitus. It generally manifests itself as an inability to adequately regulate blood-glucose levels. This is as opposed to Type I diabetes which is characterized by defects in pancreatic production of insulin. In other words, it appears that Type II diabetic individuals suffer from insulin resistance. The factors that have been identified in contributing to the development of Type II diabetes include one or more of obesity, genetic background, age, diet, and lack of exercise. Type II is frequently called "adult onset", however, because diet is a factor, it can arise at virtually any age.

The Type II diabetes can cause glucose levels to rise in the blood and urine which in turn can cause hunger, urination, thirst, and metabolism related issues. If the condition is not treated, the most common serious results include heart disease, kidney disease, and blindness. Several treatments are currently used. Because obesity is frequently a causal agent in diabetes, diet and exercise are usually a front line defense. Therapeutic agents are also used as a second line of defense including use of insulin or pharmaceuticals that reduce blood and urine levels of glucose.

Several drugs are in current use for Type II diabetes including insulin secretagogues, glucose lowering effectors, GLP-1 analogs, DPP-IV inhibitors, activators of the peroxisome proliferator activated receptor-gamma, and alpha-glucosidase inhibitors. One particular problem with the treatment with DPP-IV inhibitors is the well-known problem of the blocking through the feedback mechanism of the release of GLP-1 and related gut hormones (PYY, GLP-2, Oxyntomodulin. Because these current treatments have several problems associated with them, there is still a need for alternative therapies to treat Type II diabetes.

Gut hormones are a type of gastrointestinal hormone that, among others, cause an increase in the amount of insulin released from the beta cells of the Islets of Langerhans after eating, even before blood glucose levels become elevated. They are secreted in their highest level from L-cells in the colon. They also slow the rate of absorption of nutrients into the blood stream by reducing gastric emptying and may directly reduce food intake. They also inhibit glucagon release from the alpha cells of the Islets of Langerhans. Glucagon like peptide-1 (GLP-1), which is frequently called an incretin, is a gut hormone secreted by L cells. Glucagon like peptide-1 (GLP-1) (an incretin) has been identified as one composition that if its secretion is stimulated, can possibly be used to treat diabetes.

GLP-1 is a peptide secreted from enteroendocrine L cells and has a wide variety of physiological effects that have been described in numerous publications over the past two decades. More recently, much research has been focused on the use of GLP-1 in the treatment of conditions and disorders such as diabetes mellitus, stress, obesity, appetite control and satiety, Alzheimer's, inflammation, and diseases of the central nervous system. However, the use of a peptide in clinical treatment is severely limited due to difficult administration, and lack of sufficient in vivo stability. Therefore, a small molecule that either mimicked the effects of GLP- 1 directly, or increased GLP-1 secretion, has been thought to be the treatment of choice in increasing incretin production in the treatment of the variety of conditions or disorders described above, namely diabetes mellitus and obesity.

PYY is a gut hormone (Peptide YY) which is a short (36 amino acid) protein released by cells in the ileum and colon in response to food intake. In humans it reduces appetite. PYY is found in L-cells in the mucosa of the gastrointestinal tract especially in the ileum and colon. There is also a small amount of PYY, about 1-10 percent, in the esophagus, the stomach, the duodenum, and jejunum. PYY concentration in the circulation increases postprandially (after food ingestion) and decreases by fasting.

GLP-2 (a gut hormone) is a 33 amino acid peptide, co-secreted along with GLP-1 from intestinal endocrine cells in the small and large intestine. GLP-2, among others, stimulates mucosal growth in the small and large intestine, inhibits gastric emptying and gastric acid secretion, reduces intestinal permeability, and stimulates intestinal blood flow.

Oxyntomodulin (a gut hormone) is a 37 amino acid peptide co-secreted along with GLP-1 from L- cells that mimics the effects of GLP-1 and GLP-2 on gastric acid secretion and gut motility, suppresses appetite, and reduces food intake in normal humans, and reduces energy intake by about seventeen percent in overweight and obese human subjects with no effect on water intake.

Butyric acid is a naturally occurring fatty acid occurring in the form of esters in animal fats and plant oils. For example, the triglyceride of butyric acid makes up three percent to four percent of butter. It is found in rancid foods, such as rancid butter and rancid cheese, and has a very unpleasant smell and taste. It is an important member of the fatty acid sub-group called the short chain fatty acids.

Bile acids (also known as bile salts) are steroid acids found predominantly in the bile of mammals. In humans, taurocholic acid and glycocholic acid (derivatives of cholic acid) represent approximately eighty percent of all bile acids. The two major bile acids are cholic acid and chenodeoxycholic acid. They, their conjugates, and their 7-alpha-dehydroxylated derivatives are all found in human intestinal bile. An increase in bile flow is exhibited with an increased secretion of bile acids. Bile acid's main function is to facilitate the formation of micelles, which promotes dietary fat processing. Bile salts constitute a large family of molecules, composed of a steroid structure with four rings, a five or eight carbon side-chain terminating in a carboxylic acid, and the presence and orientation of different numbers of hydroxyl groups. The four rings are labeled from left to right (as commonly drawn) A, B, C, and D, with the D-ring being smaller by one carbon than the other three. The hydroxyl groups have a choice of being in two positions, either up (or out), termed beta (often drawn by convention as a solid line), or down, termed alpha (shown as a dashed line in drawings). All bile acids have a hydroxyl group on position three, which was derived from the parent molecule, cholesterol. In cholesterol, the four steroid rings are flat and the position of the 3-hydroxyl is beta.

Long chain fatty acids (LCFA) are fatty acids with aliphatic tails of sixteen carbons or more. Fatty acids are aliphatic monocarboxylic acids, derived from, or contained in esterified form in an animal or vegetable fat, oil, or wax. Natural fatty acids commonly have a chain of four to twenty-eight carbons (usually unbranched and even numbered), which may be saturated or unsaturated.

Glutamine is an amino acid that is used as a nutritional supplement in the treatment of a variety of diseases, including cancer. Glutamine is the most abundant free amino acid in the human body and, in addition to its role as a component of protein, serves a variety of functions in the body. It is a non-essential amino acid because it is made by body cells. In addition, most dietary proteins contain ample amounts of glutamine and healthy people usually obtain all the additional glutamine that they need in their diet.

The above naturally occurring products are difficult to administer, especially because taste of these products is extremely unpalatable and they are easily degraded in the digestive tract and/or absorbed.

Obesity is a medical condition that is reaching epidemic proportions among humans in a number of countries throughout the world. It is a condition that is also associated with, or induces, other diseases or conditions that disrupt life's activities and lifestyles. Obesity is recognized as a serious risk factor for other diseases and conditions such as diabetes, hypertension, and arteriosclerosis, and can contribute to elevated levels of cholesterol in the blood. It is also recognized that increased body weight due to obesity can place a burden on joints, such as knee joints, causing arthritis, pain, and stiffness. Obesity can contribute to certain skin conditions, such as atopic dermatitis and bed sores. Because overeating and obesity have become such a problem in the general population, many individuals are now interested in losing weight, reducing weight, and/or maintaining a healthy body weight and lifestyle.

Hypertriglyceridemia (hTG) is a common disorder in the United States. The condition is exacerbated by uncontrolled diabetes mellitus, obesity, and sedentary habits, all of which are more prevalent in industrialized societies, particularly in the United States, than in developing nations. In both epidemiologic and interventional studies, hypertriglyceridemia is a risk factor for coronary artery disease (CAD). Treatment of hypertriglyceridemia is by restriction of carbohydrates and fats in the diet, as well as with niacin, fibrates, and statins (three classes of drugs). Increased fish oil intake may substantially lower an individual's triglycerides.

There are obviously a number of compositions designed to deliver a medicament to the lower gut. Such compositions include the three-component matrix structures such as disclosed in US patent 7,431,943 to Villa et al. issued October 7, 2008 and incorporated herein in its entirety by reference.

A number of new approaches to stimulation of the receptors which appear to stimulate gut hormones, such as the GPR 120, TGR5, GPR 41, and GPR 43 receptors are being tried. In patent applications: WO/2008/067219 published June 5, 2008; US2007/060759 published November 8, 2007; JP2006-630 4A published March 9, 2006; and JP 2006-56881A published March 2, 2006 there are disclosed several classes of small molecule agonists that have been designed to stimulate the TGR5 receptor, a bile acid G-protein-coupled receptor.

A number of different formulations are available for delivery of desired compositions to the colon, including amylose coated tablets, enterically coated chitosan tablets, matrix within matrix or multimatrix systems, or polysaccharide coated tablets. One example of a multimatrix controlled release system is disclosed in US patent number 7,431,943 issued October 7, 2008 to Villa et al. and incorporated herein by reference. Disclosed is a matrix within matrix design wherein a lipophilic phase and amphiphilic phase are incorporated within the inner matrix and at least a portion of the active ingredient is incorporated into the amphiphilic phase.

Inhibitors of dipeptidyl peptidase 4 (DDP-IV) are a class of oral hypoglycemic that act by blocking DPP-IV and can be utilized to treat Type II diabetes. Their mechanism of action is believed to result from inhibition of GLP-1 degradation which in turn increases insulin secretion, decreases gastric emptying and decreases blood glucose levels. These compounds have a common drawback in that they prevent the meal-induced secretion of endogenous gut hormones. Examples of such drugs on the market or in clinical trials include sitagliptin, vildagliptin, saxagliptin, linagliptin, dutogliptin, gemigliptin, alogliptin, and berberine (berberine is an herbal dietary supplement which is known to have anti-hyperglycemic activities but is not sold for such treatment).

### BRIEF SUMMARY OF THE INVENTION

The present invention relates to the discovery that certain naturally occurring compositions can be delivered to the colon or rectally so as to bypass the stomach and upper digestive system and increase the production of certain gut hormones from L cells. This can be used to treat diabetes Type II when combined in a colon targeting formulation with inhibitors of dipeptidyl peptidase 4 (DDP-IV). These combinations are unlike, orally administrated inhibitors of dipeptidyl peptidase 4 (DDP-IV). Orally administrated DPP IV inhibitors are absorbed quickly resulting in high plasma levels, which are maintained for long time up to 24 hrs.. The composition of the present invention is selected in such a way that plasma levels of DPP IV inhibitor would not reach significant levels and therefore would allow the enzyme (DPP IV) for delayed processing of GLP-1 in plasma and would not decrease the release of gut hormones after a meal. Additionally increased levels of GLP-1 9-36, which is reported to have beneficial cardiovascular effects, will exist in systemic circulation.

In one embodiment of the present invention, there is a method of treating the condition of diabetes mellitus Type II in an individual comprising:
a) selecting an agent causing gut hormone secretion from L-cells from the group comprising butyric acid, a bile acid, a long chain fatty acid, and glutamine, the composition formulated to release in a human colon targeted delivery system or rectal release system;
b) selecting a DPP-IV inhibitor; and
c) administering to the individual the agent in combination with the DPP-IV inhibitor via a colon targeted delivery system which causes simultaneous colon release of the agent and inhibitor sufficient to cause a release of gut hormones from the L-cell in the colon of the individual wherein the inhibitor inhibits degradation of the agents.

In yet another embodiment of the present invention, there is a pharmaceutical composition for the treatment of diabetes mellitus Type II comprising;
a) a composition for inducing release of a gut hormone from an L-cell selected from the group comprising butyric acid, a bile acid, a long chain fatty acid and glutamine; and
b) a DPP-IV inhibitor;
wherein the composition and inhibitor are formulated for simultaneous delivery to the colon.

The present invention also provides, inter alia, the subject matter of the following clauses:
1. A method of treating the condition of diabetes mellitus type II in an individual comprising:
   a) selecting an agent causing gut hormone secretion from L-cells from the group comprising butyric acid, a bile acid, a long chain fatty acid and glutamine, the composition formulated to release in a human colon targeted delivery system or rectal release system;
   b) selecting a DPP-IV inhibitor; and
   c) administering the individual agent in combination with the DPP-IV inhibitor via a colon targeted delivery system which causes simultaneous colon release of the agent and inhibitor sufficient to cause a release of gut hormones from the L-cell in the colon of the individual wherein the inhibitor inhibits degradation of the agents.
2. A method according to clause 1 wherein the agent comprises glutamine.
3. A method according to clause 1 wherein agent comprises butyric acid.
4. A method according to clause 1 wherein the colon targeted delivery system is a matrix within matrix delivery system.
5. A method according to clause 4 wherein the colon targeted delivery system is a controlled release formulation of a hydrophilic first matrix comprising a lipophilic phase and an amphiphilic phase wherein the lipophilic phase and the amphiphilic phase are in a second matrix together and said second matrix is dispersed throughout the hydrophilic first matrix wherein the pharmaceutical composition is at least partially incorporated into the amphiphilic phase.
6. A method according to clause 1 wherein the targeted delivery system is simultaneously rectal administered.
7. A method according to clause 1 wherein the DPP-IV inhibitor is selected from the group consisting sitagliptin, vildagliptin, saxagliptin, linagliptin, dutogliptin, gemigliptin, alogliptin and berberine.
8. A method according to clause 7 wherein the inhibitor is vildagliptin.
9. A method according to clause 1 wherein the gut hormone from L-cells is selected from the group consisting of GLP-1, GLP-2, PYY and oxyntomodulin.
10. A pharmaceutical composition for the treatment of diabetes mellitus type 2 comprising;
   a) a composition for inducing release of a gut hormone from an L-cell selected from the group comprising butyric acid, a bile acid, a long chain fatty acid and glutamine; and
   b) a DPP-IV inhibitor;
   wherein the composition and inhibitor are formulated for simultaneous delivery to the colon.
11. A composition according to clause 10 wherein the composition is formulated in a colon targeted drug delivery system.
12. A composition according to clause 10 wherein the composition is formulated for rectal administration.
13. A composition according to clause 10 wherein the pharmaceutical composition comprises glutamine.
14. A composition according to clause 10 wherein the pharmaceutical composition comprises butyric acid.
15. A composition according to clause 10 wherein the colon targeted delivery system is a matrix within matrix system.
16. A composition according to clause 15 wherein the colon targeted delivery system is a controlled release formulation of a hydrophilic first matrix comprising a lipophilic phase and an amphiphilic phase wherein the lipophilic phase and the amphiphilic phase are in a second matrix together and said second matrix is dispersed throughout the hydrophilic first matrix wherein the pharmaceutical composition is at least partially incorporated into the amphiphilic phase.
17. A composition according to clause 10 wherein the DPP-IV inhibitor is selected from the group consisting sitagliptin, vildagliptin, saxagliptin, linagliptin, dutogliptin, gemigliptin, alogliptin and berberine.
18.A composition according to clause 11 wherein the gut hormone is selected from the group consisting of GLP-1, GLP-2, PYY and oxyntomodulin.

### DETAILED DESCRIPTION OF THE INVENTION

This detailed description defines the meaning of the terms used herein and specifically describes embodiments in order for those skilled in the art to practice the invention.

The terms "about" and "essentially" mean ±10 percent.

The term "comprising" is not intended to limit inventions to only claiming the present invention with such comprising language. Any invention using the term comprising could be separated into one or more claims using "consisting" or "consisting of" claim language and is so intended.

The terms "a" or "an", as used herein, are defined as one or as more than one. The term "plurality", as used herein, is defined as two or as more than two. The term "another", as used herein, is defined as at least a second or more. The terms "including" and/or "having", as used herein, are defined as comprising (i.e., open language). The term "coupled", as used herein, is defined as connected, although not necessarily directly, and not necessarily mechanically.

Reference throughout this document to "one embodiment", "certain embodiments", and "an embodiment" or similar terms means that a particular feature, structure, or characteristic described in connection with the embodiment is included in at least one embodiment of the present invention. Thus, the appearances of such phrases or in various places throughout this specification are not necessarily all referring to the same embodiment. Furthermore, the particular features, structures, or characteristics may be combined in any suitable manner in one or more embodiments without limitation.

The term "or" as used herein is to be interpreted as an inclusive or meaning any one or any combination. Therefore, "A, B or C" means any of the following: "A; B; C; A and B; A and C; B and C; A, B and C". An exception to this definition will occur only when a combination of elements, functions, steps or acts are in some way inherently mutually exclusive.

The term "means" preceding a present participle of an operation indicates a desired function for which there is one or more embodiments, *i.e.,* one or more methods, devices, or apparatuses for achieving the desired function and that one skilled in the art could select from these or their equivalent in view of the disclosure herein and use of the term "means" is not intended to be limiting.

As used herein, the term "treating" refers to alleviating the specified condition, eliminating or reducing the symptoms of the condition, slowing or eliminating the progression of the condition, and preventing or delaying the initial occurrence of the condition in a subject, or reoccurrence of the condition in a previously afflicted subject.

As used herein a "condition" or "disorder" refers to Diabetes Type II in a mammal such as a human or the like to which an increase in the production of a gut hormone from L cells during a meal while being treated with an inhibitor DPP IV would have a positive effect on the mammal.

The gut hormone secretion in the present invention is stimulated in L-cells present in the colon, normally in response to the presence of nutrients in the gut. This action can be partially or severely inhibited when treating diabetes Type II with an inhibitor of DPP-IV, and thus helps improve the treatment of the condition when using these type compositions. While such L-cells are present in other parts of the digestive tract and other parts of the organism, they have the highest concentration in the colon. Stimulation of L-cells in the colon results in the most effective production of gut hormones possible, and thus the most effective treatment. Gut hormones from L-cells of the present invention include, but are not limited to, GLP-1, GP-2, PYY and oxyntomodulin. Incretins such as GLP-1, in particular, are a gut hormone of interest in one embodiment.

The compounds of the invention for stimulating gut hormone release are natural compounds selected from the group comprising butyric acid, a bile acid, a long chain fatty acid, and glutamine. It is understood that this includes combinations of the compounds as well as each compound individually.

As used herein the term "inhibitors of DPP-IV" refers to compositions which are a class of oral hypoglycemic that act by blocking DPP-IV and can be utilized to treat diabetes Type II. Their mechanism of action is believed to result from high sustain levels of GLP-1 inhibiting the release of glucagon, increasing insulin secretion, decreasing gastric emptying, and decreasing blood glucose levels. These compounds have a common drawback in that they prevent the meal induced secretion of endogenous gut hormones. Examples of such drugs on the market and/or in clinical trials include sitagliptin, vildagliptin, saxagliptin, linagliptin, dutogliptin, gemigliptin, alogliptin, and berberine (berberine is an herbal dietary supplement which is known to have anti-hyperglycemic activities but is not sold for such treatment).

As used herein, "a compound" of the present invention includes all compounds described herein.

The compounds of the present invention may crystallize in more than one form, a characteristic known as "polymorphism", and such polymorphic forms ("polymorphs") are within the scope of the present invention. Polymorphism generally can occur as a response to changes in temperature, pressure, or both. Polymorphism can also result from variations in the crystallization process. Polymorphs can be distinguished by various physical characteristics known in the art such as x-ray diffraction patterns, solubility, and melting point.

Certain compounds described herein contain one or more chiral centers, or may otherwise be capable of existing as multiple stereoisomers. The scope of the present invention includes mixtures of stereoisomers as well as purified enantiomers or enantiomerically/diastereomerically enriched mixtures. Also included within the scope of the invention are the individual isomers of the compounds of the present invention, as well as any wholly or partially equilibrated mixtures, thereof. The present invention also includes the individual isomers of the compounds represented by the formulas above as mixtures with isomers, thereof, in which one or more chiral centers are inverted.

Typically, but not absolutely, the compounds herein include the salts of the present compositions and include the pharmaceutically acceptable salts. Salts encompassed within the term "pharmaceutically acceptable salts" refer to non-toxic salts of the compounds of this invention. Salts of the compounds of the present invention may include acid addition salts. Representative salts include acetate, benzenesulfonate, benzoate, bicarbonate, bisulfate, bitartrate, borate, calcium edetate, camsylate, carbonate, clavulanate, citrate, dihydrochloride, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, sulfate, tannate, tartrate, teoclate, tosylate, thethiodide, thmethylammonium, and valerate salts. Other salts, which are not pharmaceutically acceptable, may be useful in the preparation of compounds of this invention and these should be considered to form a further aspect of the invention.

"Simultaneous administering", "simultaneous release", and "simultaneous delivery" of the gut hormone from L-cells stimulating composition refers to the combination delivery of the stimulating composition and the inhibitory of DPP-IV composition to the colon at the same time. As used herein "delivery to the colon" refers to the oral or rectal administration of a composition of the present invention wherein active compositions are delivered to the colon. As described elsewhere herein, the compounds are so formulated to be taken orally and delivered to the colon bypassing the upper digestive tract and stomach or rectally to deliver the composition to the colon.

The "administering" of a composition of the present invention can refer to oral, rectal, or the like and is not dependant on any particular means of administration other than delivery to the colon as intact compositions. The inhibitor of DPP-IV is administered simultaneously with the gut hormone stimulating composition regardless of the route of administration. The inhibitor of DPP-IV is administered by the same route as the incretin stimulating composition. The amount of the inhibitor of DPP-IV administered by the present invention is an amount, which is useful to prevent the degradation of the gut hormone secreting stimulating composition with colon and until it reaches the liver. At that point DPP-IV inhibitor will be metabolized allowing normal metabolic processing of GLP-1. One skilled in the art would be able to determine the exact amount, which depends on the particular inhibitor of DPP-IV as well as the individual involved in therapy with the present invention. In the treatment of the present invention with vildagliptin of the inhibitor of DPP-IV the average dose, in one embodiment, would be from about 0.01 mg/kg to about 1 mg/kg. An effective amount of the L-cell stimulating composition is 100 mg to 2 g in a simultaneously administered composition.

As used herein, the term "effective amount" means that amount of a drug or pharmaceutical agent that will elicit the biological or medical response of a tissue, system, animal, or human that is being sought, for instance, by a researcher or clinician.

The term "therapeutically effective amount" means any amount which, as compared to a corresponding subject who has not received such an amount, results in improved treatment, healing, prevention, amelioration of a disease, disorder, side effect, or a decrease in the rate of advancement of a disease or disorder. The term also includes within its scope amounts effective to enhance normal physiological function. A therapeutically effective amount will produce a "therapeutic effect".

For use in therapy, therapeutically effective amounts of a compound of the present invention, as well as salts thereof, are presented as a pharmaceutical composition formulated to release in a colon targeted delivery system.

The present invention provides pharmaceutical compositions that include effective amounts of a compound as herein described, or a salt thereof, and one or more pharmaceutically acceptable carriers, diluents, or excipients. The carrier(s), diluent(s), or excipient(s) must be acceptable, in the sense of being compatible with the other ingredients of the formulation and not deleterious to the recipient of the pharmaceutical composition and consistent with the mode of administration i.e. oral or rectal.

In accordance with another aspect of the invention there is also provided a process for the preparation of a pharmaceutical formulation, including admixing a compound of the present invention or salts thereof, with one or more pharmaceutically acceptable carriers, diluents or excipients.

A therapeutically effective amount of a compound of the present invention will depend upon a number of factors. For example, the species, age, and weight of the recipient, the precise condition requiring treatment and its severity, the nature of the formulation, and the type of colon targeted delivery system are all factors to be considered. The therapeutically effective amount ultimately should be at the discretion of the attendant, physician, or veterinarian. Regardless, an effective amount of an incretin stimulating compound of the present invention for the treatment of humans suffering from diabetes or an overweight condition and associated conditions, generally, should be in the range of 0.01 to 100 mg/kg body weight of recipient (mammal) per day. More frequently the effective amount should be in the range of 0.3 to 30 mg/kg body weight per day. Thus, for a 70 kg adult mammal the actual amount per day would usually be from 21 to 2100 mg. This amount may be given in a single dose per day or in a number (such as two, three, four, five, or more) of sub-doses per day such that the total daily dose is the same. An effective amount of a salt or solvate thereof, may be determined as a proportion of the effective amount of the compound of the present invention per se. Similar dosages should be appropriate for treatment of the other conditions referred to herein.

Pharmaceutical formulations may be presented in unit dose forms containing a predetermined amount of active ingredient per unit dose. Such a unit may contain, as a non-limiting example, 0.5mg to 1g of an incretin stimulating compound of the present invention, depending on the condition being treated, the route of administration, and the age, weight, and condition of the patient. Preferred unit dosage formulations are those containing a daily dose or sub-dose, as herein above recited, or an appropriate fraction thereof, of an active ingredient. Such pharmaceutical formulations may be prepared by any of the methods well known in the pharmacy art.

The compounds of the present invention, or a salt thereof, are administered by a targeted drug delivery system. In one embodiment, the delivery systems may be employed for targeting drug delivery to the colon and bypassing the upper digestive system and stomach. Such drug delivery systems include covalent linkage compositions, polymer coated compositions, compositions embedded in matrices, time released compositions, redox-sensitive polymer compositions, bioadhesive compositions, micropartical coating compositions, and osmotic delivery compositions. Suitable compositions include those containing polysaccharides, such as chitosan, pectin, chondroitin sulphate, cyclodexthn, dextrans, guar gum, inulin, amylase, and locust bean gum. The compounds may also be coupled with soluble polymers. Such polymers can include polyvinylpyrrolidone (PVP), pyran copolymer, polyhydroxypropylmethacrylamide-phenol, polyhydroxyethyl-aspartamidephenol, or polyethyleneoxidepolylysine substituted with palmitoyl residues. Furthermore, the compounds may be coupled to a class of biodegradable polymers; for example, polylactic acid, polyepsilon caprolactone, polyhydroxy butyric acid, polyorthoesters, polyacetals, polydihydropyrans, polycyanoacrylates, and cross-linked or amphipathic block copolymers of hydrogels. Those of particular effectiveness in the present invention include embodiments of multimatrix targeted systems. Of particular effectiveness in the present invention are the targeted matrix-in-matrix systems comprising a formulation of a hydrophilic first matrix, comprising a lipophlic phase and an amphiphilic phase, wherein the liphphilic phase and the amphiphilic phase are in a second matrix together and the second matrix is dispersed throughout the hydrophilic first matrix and wherein the pharmaceutical composition containing the compound is at least partially incorporated into the amphiphilic phase. Examples of some of the matrix-in-matrix formulations are disclosed in US patent 7,431,943 as noted above. Those skilled in the art will appreciate the use of such compositions for the purposes of targeting delivery of the compounds of the present invention, or a salt thereof, to the colon of the subject being treated. The methods for the formulation of such compositions for targeted delivery are within the skill of the art, in view of this disclosure.

The compounds of the present invention, or a salt thereof, may be employed alone or in combination with other therapeutic agents. In one embodiment they are combined with other agents useful for the treatment of diabetes Type II. The compound(s) of the present invention and the other pharmaceutically active agent(s) may be administered together or separately and, when administered separately, administration may occur simultaneously or sequentially, in any order. The amounts of the compound(s) of the present invention and the other pharmaceutically active agent(s) and the relative timings of administration will be selected in order to achieve the desired combined therapeutic effect. The administration in combination of a compound of the present invention or a salt, or solvate thereof, with other treatment agents may be in combination by administration concomitantly in: (1) a unitary pharmaceutical composition including both compounds; or (2) separate pharmaceutical compositions each including one of the compounds. Alternatively, the combination may be administered separately in a sequential manner wherein one treatment agent is administered first and the other second or vice versa. Such sequential administration may be close in time or remote in time.

The compounds may also be formulated in rectal compositions such as suppositories or retention enemas, e.g. containing conventional suppository bases such as cocoa butter or other glycerides. The compositions so formulated will be designed to give an effective dosage to the colon in addition to other areas a rectal administration might affect.

The compounds of the present invention may be used in the treatment of diabetes Type II. As such, the compounds of the present invention may be used in combination with a variety of other therapeutic agents useful in the treatment of this condition. As discussed briefly above, current diabetes therapies include diet, exercise, insulin, insulin secretagogues, glucose-lowering effectors, PPAR-γ agonists, and α-glucosidase inhibitors. The compounds of the present invention may be combined with these or other medical therapies to treat and/or prevent diabetes and associated disorders and conditions, including but not limited to, diabetes Types I and II, obesity, glucose intolerance, insulin resistance, metabolic syndrome, hyperlipidemia, hypercholesterolemia, artheroscelrosis, neurodegenerative diseases, and other indications such as inflammation and stroke.

### EXAMPLES

### EXAMPLE 1

Drug is delivered as an enema or suppositories made as described in¹ (containing 1g of glutamine). Ten overnight fasted diabetic Type II patients are dosed rectally with one suppository (or enema). Thirty minutes after drug administration, patients are subjected to Oral Glucose Tolerance Test (OGTT) or standardized meal. Blood is collected at the following time points: -30, 0, 5; 10, 15, 30, 60, 90, and 120 minutes. Blood is analyzed for levels of: glucose, insulin, GLP-1, PYY, other hormones and lipids. Glucose and lipids (after standardized meal) levels are measured after treatment regime and shown to decrease.

### EXAMPLE 2

Drug is delivered as an enema or suppositories made as described in¹ (containing 2g of butyric acid). Ten overnight fasted diabetic Type II patients are dosed rectally with one suppository (or enema). Thirty minutes after drug administration, patients are subjected to Oral Glucose Tolerance Test (OGTT) or standardized meal. Blood is collected at the following time points: -30, 0, 5; 10, 15, 30, 60, 90, and 120 minutes. Blood is analyzed for levels of: glucose, insulin, GLP-1, PYY, other hormones and lipids. Glucose and lipids' (after standardized meal) levels are measured after treatment regime and their levels are shown to decrease.

### EXAMPLE 3

Tablets formulated with MMX technology (containing 1g of glutamine) are made as described in². Ten overnight fasted diabetic Type II patients with are dosed with one MMX tablet at 8:00 AM. Four hours after drug administration, patients are subjected to Oral Glucose Tolerance Test (OGTT) or standardized meal. Blood is collected at the following time points: -30, 0, 5; 10, 15, 30, 60, 90, and 120 minutes. Blood is analyzed for levels of: glucose, insulin, GLP-1, PYY, other hormones and lipids. Glucose and lipids' (after standardized meal) levels are measured after treatment regime and their levels are shown to decrease.

### EXAMPLE 4

Tablets formulated with MMX technology (containing 2g of butyric acid) are made as described in². Ten overnight fasted diabetic Type II patients are dosed with one MMX tablet at 8:00 AM. Four hours after drug administration, patients are subjected to Oral Glucose Tolerance Test (OGTT) or standardized meal. Blood is collected at the following time points: -30, 0, 5; 10, 15, 30, 60, 90, and 120 minutes. Blood is analyzed for levels of: glucose, insulin, GLP-1, PYY, other hormones, and lipids. Glucose and lipids' (after standardized meal) levels are measured after treatment regime and their levels are shown to decrease.

### EXAMPLE 5

Tablets formulated with MMX technology (containing 1g of glutamine) are made as described in². Ten diabetic Type II patients are dosed with one MMX tablet at 8:00 AM before first meal for six (6) weeks. HbA1c, fasting glucose and insulin are measured before treatment and at 1, 2, and 6 weeks after initiation of the treatment. Additionally, patients are subjected at these times to Oral Glucose Tolerance Test (OGTT) or standardized meal. Blood is collected at the following time points: -30, 0, 5; 10, 15, 30, 60, 90, and 120 minutes. Blood is analyzed for levels of: glucose, insulin, GLP-1, PYY, other hormones, and lipids. Glucose levels are measured to decrease after treatment regime. The treatment also caused triglycerides levels to be lower than pre-treatment levels.

### EXAMPLE 6

Each of the above examples are combined with vildagliptin (Galvus, Novartis) 5mg in one (1) timed released tablet for once a day dosing (MMX). Diabetes Type II patients are treated with the combination therapy and again glucose disposal in OGTT is tested in acute experiment and HbA1c levels are measured in chronic experiment. It is observed that the combination therapy results in an improved diabetes Type II condition when compared with the condition alone in that either lower doses of drugs, comparing to use of single agent, are required for the same efficacy or using the same amounts as in single drug therapy, higher efficacy is achieved as measured by lowering of HbA1c after chronic dosing.

The following references are included in the application by reference in their entirety.
1. Mayo Clin. Proc. 1993, Vol 68, 978 incorporated herein by reference,
2. US Patent 7,431,943 B1 incorporated herein by reference,
3. Diabetes, Obesity and Metabolism, 9 (Suppl. 1), 2007, 23-31 incorporated herein by reference,
4. Toft-Nielsen MB, Damholt MB, Madsbad S et al. Determinants of the impaired secretion of glucagon-like peptide-1 in type 2 diabetic patients. J Clin Endocrinol Metab 2001;86:3717-3723,
5. Rask E, Olsson T, Soderberg S et al. Impaired incretin response after a mixed meal is associated with insulin resistance in nondiabetic men. Diabetes Care 2001;24:1640-1645,
6. Provisional patent applications (BIOK001PR) 61/143,951 filed January 12, 2009 and (BIOK001PR-C) 61/293,773 filed January 11, 2010 incorporated herein in their entirety by reference, and
7. BIOK001-C-PCT application number PCT/US2010/020629 incorporated herein in its entirety by reference.

## Claims

1. A pharmaceutical composition suitable for the treatment of diabetes mellitus type II, the composition comprising:
a) an agent for inducing release of a gut hormone from an L-cell, wherein the agent is butyric acid; and
b) a DPP-IV inhibitor;
wherein the agent and inhibitor are formulated for simultaneous delivery to the colon.

2. A pharmaceutical composition for use in the treatment of an individual with diabetes mellitus type II,
wherein the composition comprises:
a) an agent for inducing release of a gut hormone from an L-cell , wherein the agent is butyric acid; and
b) a DPP-IV inhibitor;
wherein the agent and inhibitor are formulated for simultaneous delivery to the colon.

3. The composition according to claim 1 or the composition for use in treatment according to claim 2, wherein the composition is formulated in a colon targeted drug delivery system.

4. The composition according to claim 1 or the composition for use in treatment according to claim 2, wherein the composition is formulated for rectal administration.

5. A composition for use in the treatment of the condition of diabetes mellitus type II in an individual, the composition comprising an agent causing gut hormone secretion from L-cells, wherein the agent is butyric acid, the composition being formulated to release in a human colon targeted delivery system or rectal release system,
wherein the composition is for use in a method of treating the condition of diabetes mellitus type II that comprises:
a) providing the composition comprising the agent causing gut hormone secretion from L-cells;
b) selecting a DPP-IV inhibitor; and
c) administering the agent in combination with the DPP-IV inhibitor via a colon targeted delivery system which causes simultaneous colon release of the agent and inhibitor sufficient to cause a release of gut hormones from the L-cell in the colon of the individual wherein the inhibitor inhibits degradation of the gut hormones.

6. A composition for use in the treatment of the condition of diabetes mellitus type II in an individual, the composition comprising a DPP-IV inhibitor,
wherein the composition is for use in a method of treating the condition of diabetes mellitus type II that comprises:
a) selecting an agent causing gut hormone secretion from L-cells, wherein the agent is butyric acid, and providing the agent in a composition formulated to release in a human colon targeted delivery system or rectal release system;
b) providing the composition comprising the DPP-IV inhibitor; and
c) administering the individual agent in combination with the DPP-IV inhibitor via a colon targeted delivery system which causes simultaneous colon release of the agent and inhibitor sufficient to cause a release of gut hormones from the L-cell in the colon of the individual wherein the inhibitor inhibits degradation of the gut hormones.

7. A composition for use in the treatment of the condition of diabetes mellitus type II in an individual, the composition comprising (i) an agent causing gut hormone secretion from L-cells, wherein the agent is butyric acid, in combination with (ii) a DPP-IV inhibitor, the composition being formulated to release in a human colon targeted delivery system or rectal release system,
wherein the composition is for use in a method of treating the condition of diabetes mellitus type II that comprises administering the agent in combination with the DPP-IV inhibitor via a colon targeted delivery system which causes simultaneous colon release of the agent and inhibitor sufficient to cause a release of gut hormones from the L-cell in the colon of the individual wherein the inhibitor inhibits degradation of the gut hormones.

8. The composition for use in treatment according to any one of claims 5-7,
wherein the targeted delivery system is simultaneously rectally administered.

9. The composition or the composition for use in treatment according to any one of claims 1-8, wherein the colon targeted delivery system is a matrix within matrix delivery system.

10. The composition or the composition for use in treatment according to claim 9,
wherein the colon targeted delivery system is a controlled release formulation of a hydrophilic first matrix comprising a lipophilic phase and an amphiphilic phase wherein the lipophilic phase and the amphiphilic phase are in a second matrix together and said second matrix is dispersed throughout the hydrophilic first matrix wherein the pharmaceutical composition is at least partially incorporated into the amphiphilic phase.

11. The composition or the composition for use in treatment according to any one of claims 1-10, wherein the DPP-IV inhibitor is selected from the group consisting sitagliptin, vildagliptin, saxagliptin, linagliptin, dutogliptin, gemigliptin, alogliptin and berberine.

12. The composition or the composition for use in treatment according to claim 11, wherein the inhibitor is vildagliptin.

13. The composition or the composition for use in treatment according to any one of claims 1-12, wherein the gut hormone from L-cells is selected from the group consisting of GLP-1, GLP-2, PYY and oxyntomodulin.

14. The composition or the composition for use in treatment according to any one of claims 1-13, wherein the composition is formulated:
i. to be taken orally and delivered to the colon, bypassing the upper digestive tract and stomach; or
ii. to be administered rectally, to deliver the composition to the colon.
